# EUROPÄISCHE PATENTANMELDUNG

(11) **EP 2 005 991 A1**
(43) Veröffentlichungstag der Anmeldung: **24.12.2008**
(21) Anmeldenummer: 08158597.8
(22) Anmeldetag: 19.06.2008
(51) Int. Cl.: A61N 5/06

(54) **Einrichtung für das Bestrahlen eines menschlichen Körpers mit UV-Licht nebst Verfahren**

(30) Priorität: 20.06.2007 DE 102007028808
(71) Anmelder: Hinte, Dirk, 45136 Essen (DE)
(72) Erfinder: Hinte, Dirk, 45136 Essen (DE)
(74) Vertreter: Gille Hrabal Struck Neidlein Prop Roos

(57) **Zusammenfassung**

Die Erfindung betrifft eine Einrichtung für das Bestrahlen eines menschlichen Körpers mit UV-Licht, die auch Solarium oder Sonnenstudio genannt wird, nebst Verfahren.

Es ist Aufgabe der Erfindung, eine Einrichtung nebst verfahren für das Bestrahlen eines menschlichen Körpers mit UV - Licht bereit zu stellen, die mit einer geringen Grundfläche auskommt und die komfortabler im Vergleich zu einer Stehkabine ist.

Zur Lösung der Aufgabe ist die Einrichtung für das Bestrahlen eines menschlichen Körpers zunächst einmal wie eine Stehkabine ausgebildet. Die Stehkabine weist eine relativ kleine Stellfläche im Vergleich zu ihrer Höhe auf. UV - Strahler sind in der Stehkabine so angeordnet, dass eine gleichmäßige Bestrahlung eines menschlichen Körpers möglich ist. Im Unterschied zu einer vorbekannten Stehkabine umfasst die Einrichtung eine Sitzfläche. Diese Sitzfläche ist so angeordnet oder kann so bewegt werden, dass ein Mensch auf dieser sitzen kann. Dies verbessert den Komfort gegenüber dem bekannten Stand der Technik.

## Beschreibung

Die Erfindung betrifft eine Einrichtung für das Bestrahlen eines menschlichen Körpers mit UV-Licht, die auch Solarium oder Sonnenstudio genannt wird, Eine solche Einrichtung ist aus der Druckschrift DE 102004036353 Al bekannt.

Eine Einrichtung für das Bestrahlen eines menschlichen Körpers umfasst eine Mehrzahl von UV - Strahler. Diese UV - Strahler werden in einer solchen Einrichtung verteilt um einen menschlichen Körper angeordnet, um eine gleichmäßige Ganzkörperbräunung erreichen zu können, Aus der Druckschrift DE 102004036353 Al ist bekannt, eine solche Einrichtung als Stehkabine auszubilden und dann spezielle Gesichts-, Bein- und/ oder Schulterbräuner vorzusehen.

Regelmäßig werden Liegen oder Liegekabinen eingesetzt, die mit UV - Strahlern versehen sind, um einen menschlichen Körper vollständig mit UV - Licht zu bestrahlen. Auf einer Liege zu liegen, ist für einen Menschen besonders komfortabel. Allerdings nehmen Liegen vergleichsweise viel Grundfläche ein, Soll Grundfläche und damit Kosten eingespart werden, so sind Stehkabinen zu bevorzugen. Das Stehen in einer Stehkabine ist allerdings für einen Menschen deutlich weniger komfortabel.

Es ist Aufgabe der Erfindung, eine Einrichtung nebst einem zugehörigen Verfahren für das Bestrahlen eines menschlichen Körpers mit UV - Licht bereit zu stellen, die mit einer geringen Grundfläche auskommt und die komfortabler im Vergleich zu einer Stehkabine ist.

Die Aufgabe der Erfindung wird mit einer Einrichtung mit den Merkmalen des Anspruchs 1 gelöst. Vorteilhafte Ausgestaltungen ergeben sich aus den Unteransprüchen.

Zur Lösung der Aufgabe ist die Einrichtung für das Bestrahlen eines menschlichen Körpers zunächst einmal wie eine Stehkabine ausgebildet. Die Stehkabine weist eine Stellfläche mit relativ kleinem Durchmesser im Vergleich zu ihrer Höhe auf. UV - Strahler sind in der Stehkabine seitlich so angeordnet, dass eine gleichmäßige Bestrahlung eines menschlichen Körpers möglich ist, Im Unterschied zu einer vorbekannten Stehkabine umfasst die Einrichtung eine Sitzfläche. Diese Sitzfläche ist so angeordnet oder kann so bewegt werden, dass ein Mensch auf dieser sitzen kann. Dies verbessert den Komfort gegenüber dem bekannten Stand der Technik.

Der Fachmann hat von einer solchen Sitzfläche bisher abgesehen, da eine Sitzfläche eine vollständige Bräunung eines menschlichen Körpers behindert. Um diesen Nachteil auszugleichen, ist die Sitzfläche in einer Ausgestaltung der Erfindung aus einem Material gefertigt, welches durchlässig für UV - Strahlen ist. In Betracht kommt beispielsweise ein durchsichtiger Kunststoff. Vorzugsweise sind die Mittel, die die Sitzfläche in der gewünschten Position halten, ebenfalls aus einem Material gefertigt, welches UV - Strahlen durchlässt. Auf diese Weise wird weiter verbessert vermieden, dass ein menschlicher Körper ungleichmäßig gebräunt wird.

Allerdings wird allein die Wahl des Materials in der Regel nicht ausreichen, um eine gleichmäßige Bräunung des menschlichen Körpers sicherzustellen, wenn ein Mensch auf der Sitzfläche sitzt. In einer Ausgestaltung der Erfindung umfasst die Einrichtung daher unterhalb der Sitzfläche einen UV - Strahler, der so gerichtet ist oder so ausgerichtet werden kann, dass UV Strahlen gezielt durch die Sitzfläche hindurch gelenkt werden. So wird die gleichmäßige Bräunung eines menschlichen Körpers besonders zuverlässig erreicht,

In einer Ausgestaltung der Erfindung ist die Sitzfläche in der Kabine schwenkbar angeordnet. Die Sitzfläche kann weg geklappt werden, um Raum für das Betreten der Kabine bereitzustellen. Da die Stellfläche der Kabine gering sein soll, ist es zweckmäßig, einen Schwenkmechanismus für die Sitzfläche vorzusehen, um mit einer minimalen Stellfläche für die Kabine auskommen zu können.

In einer weiteren Ausgestaltung der Erfindung ist der UV - Strahler, der UV - Licht von unten durch die Sitzfläche hindurch zu strahlen vermag, ebenfalls schwenkbar angeordnet. Soll die Sitzfläche nicht verwendet werden, so kann dieser UV - Strahler für die Nichtbenutzung weg geschwenkt werden,

In einer Ausgestaltung der Erfindung ist der UV - Strahler, der UV - Licht von unten durch die Sitzfläche hindurch zu strahlen vermag, so ausgestaltet, dass ein Wegschwenken im vorgenannten Sinne zur Konsequenz hat, dass der UV - Strahler ausgeschaltet wird und bleibt. Fehlbedienungen werden so vermieden.

Nachfolgend wird die Erfindung anhand eines Ausführungsbeispiels näher erläutert.

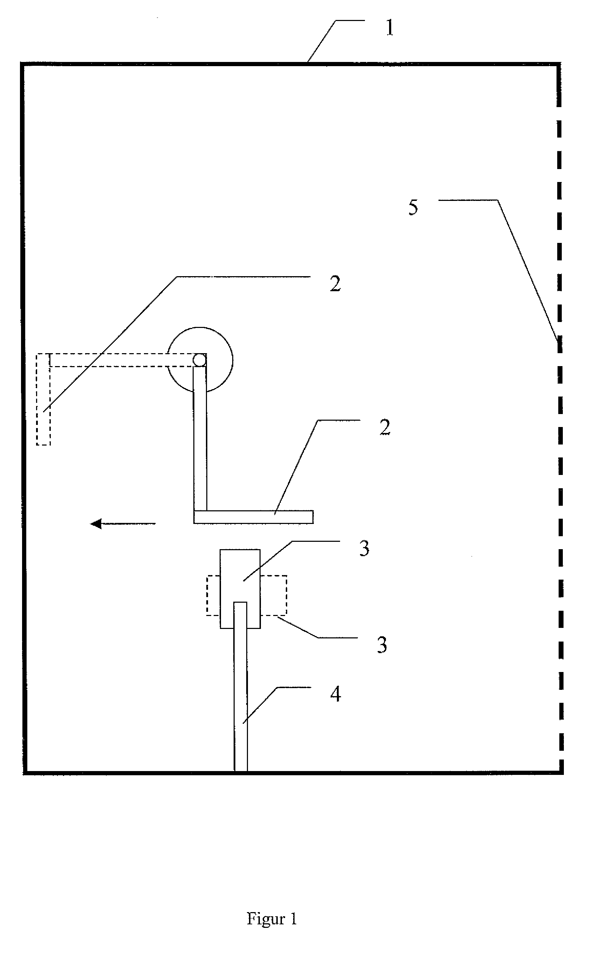

Figur 1 zeigt im Schnitt eine Stehkabine 1, die in bekannter Weise mit UV-Strahlern versehen ist, um eine Ganzkörperbräunung erzielen zu können. Zusätzlich ist eine Sitzfläche 2 schwenkbar in der Kabine angebracht. Die gestrichelte Darstellung des Sitzes 2 zeigt die Sitzfläche 2 im hochgeschwenkten Zustand, um das Betreten der Kabine zu erleichtern.

Um den in der Kabine zur Verfügung stehenden Raum verbessert auszunutzen, ist in einer Ausgestaltung der Erfindung der Sitz zusätzlich verfahrbar, um diesen nach links (Pfeilrichtung) verfahren zu können.

Unterhalb der Sitzfläche 2 befindet sich ein zusätzlicher LJv-Strahler 3, der auf dem Boden der Kabine mittels einer Säule 4 steht, und seine LJV-Strahlung nach oben in Richtung Sitzfläche 2 abzugeben vermag. Die Anordnung mittels einer Säule ermöglicht es, den UV-Strahler 3 über die Säule mit Strom zu versorgen. Die elektrischen Leitungen werden so vor einem unerwünschten Zugriff einerseits geschützt. Auch optisch ist dies vorteilhaft. Der UV-Strahler 3 kann ebenfalls weggeklappt werden, wie die gestrichelte Darstellung andeutet. In dieser Stellung wird der Strahler zugleich dauerhaft ausgeschaltet und zwar unabhängig von einer davon getrennten Ausschaltmöglichkeit. In einer Ausgestaltung der Erfindung ist die Säule 4 ebenfalls verfahrbar ausgestaltet, so zum Beispiel mittels einer im Kabinenboden eingelassenen Schiene, um so weiter den zur Verfügung stehenden Raum maximal ausnutzen zu können.

Die Kabine verfügt außerdem über eine Tür 5, um die Kabine hierüber betreten und verschließen zu können.

Die Strahlungsintensitäten der einzelnen UV-Strahler können getrennt voneinander eingestellt werden, um so die Bestrahlung derart regeln zu können, dass eine gleichmäßige Bräunung eines menschlichen Körpers erzielt werden kann.

## Patentansprüche

1. Einrichtung für das Bestrahlen eines menschlichen Körpers mit UV-Licht mit einer Kabine (1) sowie mit einer Mehrzahl an UV-Strahlern, wobei die Kabine (1) als Stehkabine ausgestaltet ist, **dadurch gekennzeichnet, dass** die Kabine eine Sitzfläche (2) aufweist.

2. Einrichtung nach Anspruch 1, **dadurch gekennzeichnet, dass** die Sitzfläche (2) aus einem für UV-Licht durchlässigen Material besteht.

3. Einrichtung nach Anspruch 2, **dadurch gekennzeichnet, dass** unterhalb der Sitzfläche (2) ein UV - Strahler (3) angeordnet ist, der seine Strahlung in Richtung Sitzfläche abzugeben vermag.

4. Einrichtung nach einem der vorhergehenden Ansprüche, **dadurch gekennzeichnet, dass** die Sitzfläche (2) schwenkbar in der Kabine (1) untergebracht ist.

5. Einrichtung nach einem der vorhergehenden Ansprüche, **dadurch gekennzeichnet, dass** ein unterhalb der Sitzfläche angeordneter UV - Strahler (2) verfahrbar in der Kabine (1) angeordnet ist.

6. Einrichtung nach einem der vorhergehenden Ansprüche, **dadurch gekennzeichnet, dass** ein unterhalb der Sitzfläche angeordneter UV - Strahler mittels einer Säule (4) auf dem Boden der Kabine aufgestellt ist.

7. Verfahren zur Bräunung einer menschlichen Haut mittels einer Einrichtung nach einem der vorhergehenden Ansprüche, indem ein Mensch sich auf die Sitzfläche (2) setzt und durch die UV-Strahler bestrahlt wird.
